# EUROPÄISCHE PATENTANMELDUNG

(11) **EP 1 074 242 A2**
(43) Veröffentlichungstag der Anmeldung: **07.02.2001**
(21) Anmeldenummer: 00115432.7
(22) Anmeldetag: 18.07.2000
(51) Int. Cl.: A61K 7/48

(54) **Verwendung von kosmetischen oder dermatologischen Zubereitungen zur Stabilisierung von Ascorbinsäure und/oder Ascorbylverbindungen**

(30) Priorität: 04.08.1999 DE 19936686
(71) Anmelder: Beiersdorf Aktiengesellschaft, 20245 Hamburg (DE)
(72) Erfinder: Raschke, Thomas, Dr., 25421 Pinneberg (DE); Rapp, Claudius, Dr., 20257 Hamburg (DE)

(57) **Zusammenfassung**

Verwendung von kosmetischen oder dermatologischen Zubereitungen, enthaltend
(I) einen oder mehrere partiell neutralisierte Ester von Monoglyceriden und/oder Diglyceriden gesättigter Fettsäuren,
(II) einen oder mehrere Ester von Polyethylenglykol mit verzweigten und/oder unverzweigten, gesättigten und/oder ungesättigten Fettsäuren einer Kettenlänge von 12 bis 40 Kohlenstoffatomen,
(III) mindestens zwei Polyethylenglykolether A und B, welche unabhängig voneinander gewählt werden aus der Gruppe der Ether von Polyethylenglykol mit verzweigten und/oder unverzweigten, gesättigten und/oder ungesättigten Fettalkoholen einer Kettenlänge von 16 bis 40 Kohlenstoffatomen, wobei sich die Kettenlänge des Ethers A um mindestens zwei Kohlenstoffatome von der Kettenlänge des Ethers B unterscheidet,
(IV) einen oder mehrere Fettalkohole, gewählt aus der Gruppe verzweigten und/oder unverzweigten, gesättigten und/oder ungesättigten Alkylalkohole mit 12 bis 40 Kohlenstoffatomen,
zur Stabilisierung von Ascorbinsäure und/oder Ascorbylverbindungen.

## Beschreibung

Die vorliegende Erfindung betrifft die Verwendung von kosmetischen oder dermatologischen Zubereitungen zur Stabilisierung von Ascorbinsäure und/oder Ascorbylverbindungen. Bevorzugt betrifft die vorliegende Erfindung kosmetische oder dermatologische Zubereitungen, in welchen Ascorbinsäure und/oder Ascorbylverbindungen über einen langen Zeitraum stabil gelagert werden können und welche ein gutes Transportmittel für diese Wirkstoffe darstellen.

L-Ascorbinsäure {(R)-5-[(S)-1,2-Dihydroxyethyl]-3,4-dihydroxy-5-H-furan-2-on, auch unter der Bezeichnung Vitamin C bekannt} zeichnet sich durch die Strukturformel 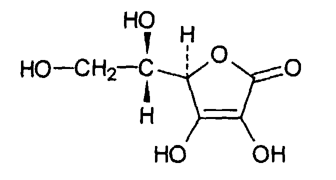 aus. Ascorbinsäure ist leicht löslich in Wasser (sowohl in der Säureform als auch als Salz) und gut löslich in Alkohol (dagegen unlöslich in Fetten und Ölen). Ascorbinsäure ist ein Endiol und wirkt als Reduktionsmittel stark reduzierend. Ascorbinsäure ist wärmeempfindlich und wird insbesondere in Gegenwart von Schwermetallspuren sowie in alkalischem Milieu durch Licht und Luftsauerstoff zersetzt, in reinem, trockenem Zustand ist sie dagegen relativ beständig gegen Licht, Luft und Wärme.

Vitamin C ist seit langem für seinen positiven Einfluß auf das Erscheinungsbild der Haut bekannt. So wird durch Ascorbinsäure beipielsweise die Produktion von Kollagen im menschlichen Hautbindegewebe angeregt. Kollagen ist das für die Haut wichtigste Protein. Es gehört zu den Faserproteinen, ist wesentlich für die Festigkeit des Gewebes und zeichnet sich durch eine hohe Wasserbindungsfähigkeit aus. Eine Erhöhung des Kollagengehaits der Haut führt zu einer besseren Feuchtigkeitsstabilisierung des Stratum Corneum, wodurch letztendlich Falten und Fältchen gemildert werden können.

Aus der WO-Schrift WO 98/00103-A1 geht hervor, daß Vitamin C ferner möglicherweise auch eine UV-absorbierende Wirkung hat. Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. UV-A-Strahlung (320 bis 400 nm) ist im Hinblick auf die Auslösung photodynamischer, speziell phototoxischer Reaktionen und chronischer Veränderungen der Haut noch weitaus gefährlicher als UV-B-Strahlung. So reicht selbst die UV-A-Strahlung unter ganz normalen Alltagsbedingungen aus, um innerhalb kurzer Zeit die Kollagen- und Elastinfasern zu schädigen. Auch kann der schädigende Einfluß der UV-B-Strahlung durch UV-A-Strahlung noch verstärkt werden.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können kosmetischen bzw. dermatologischen Formulierungen zusätzlich Antioxidantien und/oder Radikalfänger zugesetzt werden.

Ascorbinsäure gehört darüberhinaus auch zur Gruppe der Antioxidantien. Antioxidantien sind Stoffe, welche Oxidationsprozesse verhindern. Sie sind chemische Substanzen unterschiedlichster Struktur und hemmen oder unterbinden unerwünschte, durch Sauerstoff-Einwirkungen, UV-Strahlung und andere oxidative Prozesse bedingte Veränderungen in den zu schützenden Stoffen. Antioxidantien werden insbesondere eingesetzt, um organische Produkte zu schützen, namentlich beispielsweise Fette und Öle, aber auch z. B. kosmetische und dermatologische Wirk- und Zusatzstoffe wie beispielsweise Aromastoffe und dergleichen. Lipide mit Doppelbindungen, insbesondere ungesättigte Fettsäuren, unterliegen unter normalen Lagerbedingungen einem Verderb, der auch als Ranzigkeit bezeichnet wird. Die dadurch hervorgerufenen Veränderungen bewirken eine deutliche Beeinflussung der organoleptischen und anwendungsbezogenen Eigenschaften, im Extremfall kann sich sogar die dermatologische Verträglichkeit eines Rohstoffes drastisch verschlechtern. Diesem Prozeß kann durch Zusatz von Antioxidantien entgegengewirkt werden.

Die kosmetische Hautpflege dient in erster Linie dazu, die natürliche Funktion der Haut als Barriere gegen Umwelteinflüsse und gegen den Verlust von körpereigenen Stoffen (neben Wasser auch natürliche Fette, Elektrolyte etc.) zu stärken oder wiederherzustellen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycenn, Glykole usw.) bestehen, die mit Hilfe von Emulgatoren in Form feinster Tröpfchen ineinander verteilt werden. Liegt die Ölphase fein verteilt in der Wasserphase vor, so handelt es sich um eine Öl-in-Wasser-Emulsion (O/W-Emulsion, z. B. Milch). Der Grundcharakter einer O/W-Emulsion ist durch das Wasser geprägt. Im Gegensatz zu einer W/O-Emulsion wirkt sie daher weniger fettend auf der Haut, ist eher mattierend und zieht schneller in die Haut ein. O/W-Emulsionen werden aufgrund dieser sensorischenVorteile für Cremes und Lotionen verwendet, die keinen sichtbaren Fettglanz auf der Haut hinterlassen sollen, wie beispielsweise für Tages- (Gesichts-)cremes und -lotionen.

Aus dem bisher gesagten geht hervor, daß der Einsatz von Vitamin C und/oder seinen Derivaten in kosmetischen und dermatologischen Formulierungen, insbesondere in Formulierungen vom Typ Öl-in-Wasser, sehr wünschenswert ist. Unglücklicherweise ist Vitamin C aber eine sehr instabile Substanz, welche insbesondere in wäßrigen Medien schnell zerfällt. Da aber gerade O/W-Formulierungen in diesem Zusammenhang als wäßrige Systeme anzusehen sind, stellt die Einarbeitung von Vitamin C und/oder seinen Derivaten in diesen Formulierungstyp bisher ein erhebliches Problem dar. Da diese Wirkstoffe darüberhinaus schlecht ällöslich ist, läßt sich auch in nicht-wäßrigen Systemen nur schwer eine wirksame Konzentration an Vitamin C erreichen.

In kosmetischen und dermatologischen Zubereitungen werden anstatt der Ascorbinsäure oftmals auch Ascorbylverbindungen, bevorzugt Ascorbylester von Fettsäuren, besonders bevorzugt Ascorbylpalmitat eingesetzt, da die Empfindlichkeit dieser Verbindungen auf oxidativen Einfluß gegenüber der Ascorbinsäure herabgesetzt ist und diese Verbindungen zumeist besser öllöslich sind, was galenische Vorteile bieten kann. Allerdings steht auch hier die erzielte Wirkung weit hinter der gewünschten zurück.

Aufgabe der vorliegenden Erfindung war es daher, die Nachteile des Standes der Technik zu beseitigen und Ascorbylverbindungen, insbesondere Vitamin C und Vitamin-C-Ester gegen schädlichen oxidativen Einfluß zu schützen, und zwar bevorzugt in kosmetischen oder dermatologischen Zubereitungen. Ferner war Aufgabe, kosmetische oder dermatologische Zubereitungen zu finden, in welchen Ascorbinsäure und/oder Ascorbylverbindungen über einen langen Zeitraum stabil gelagert werden können und welche ein gutes Transportmittel für diese Wirkstoffe darstellen.

Es war indes überraschend und für den Fachmann nicht vorherzusehen, daß die Verwendung von kosmetischen oder dermatologischen Zubereitungen, enthaltend
(I) einen oder mehrere partiell neutralisierte Ester von Monoglyceriden und/oder Diglyceriden gesättigter Fettsäuren,
(II) einen oder mehrere Ester von Polyethylenglykol mit verzweigten und/oder unverzweigten, gesättigten und/oder ungesättigten Fettsäuren einer Kettenlänge von 12 bis 40 Kohlenstoffatomen,
(III) mindestens zwei Polyethylenglykolether A und B, welche unabhängig voneinander gewählt werden aus der Gruppe der Ether von Polyethylenglykol mit verzweigten und/oder unverzweigten, gesättigten und/oder ungesättigten Fettalkoholen einer Kettenlänge von 16 bis 40 Kohlenstoffatomen, wobei sich die Kettenlänge des Ethers A um mindestens zwei Kohlenstoffatome von der Kettenlänge des Ethers B unterscheidet und
(IV) einen oder mehrere Fettalkohole, gewählt aus der Gruppe verzweigten und/oder unverzweigten, gesättigten und/oder ungesättigten Alkylalkohole mit 12 bis 40 Kohlenstoffatomen
zur Stabilisierung von Ascorbinsäure und/oder Ascorbylverbindungen, den Nachteilen des Standes der Technik abhelfen.

Gegenstand der Erfindung sind ferner kosmetische und dermatologische Zubereitungen, enthaltend
(I) einen oder mehrere partiell neutralisierte Ester von Monoglyceriden und/oder Diglyceriden gesättigter Fettsäuren,
(II) einen oder mehrere Ester von Polyethylenglykol mit verzweigten und/oder unverzweigten, gesättigten und/oder ungesättigten Fettsäuren einer Kettenlänge Von 12 bis 40 Kohlenstoffatomen,
(III) mindestens zwei Polyethylenglykolether A und B, welche unabhängig voneinander gewählt werden aus der Gruppe der Ether von Polyethylenglykol mit verzweigten und/oder unverzweigten, gesättigten und/oder ungesättigten Fettalkoholen einer Kettenlänge von 16 bis 40 Kohlenstoffatomen, wobei sich die Kettenlänge des Ethers A um mindestens zwei Kohlenstoffatome von der Kettenlänge des Ethers B unterscheidet,
(IV) einen oder mehrere Fettalkohole, gewählt aus der Gruppe verzweigten und/oder unverzweigten, gesättigten und/oder ungesättigten Alkylalkohole mit 12 bis 40 Kohlenstoffatomen
(V) einen oder mehrere Verdicker, gewählt aus der Gruppe der Xanthangummies und
(VI) eine oder mehrere Verbindungen gewählt aus der Gruppe bestehend aus Ascorbinsäure und Ascorbylverbindungen.

Ascorbylverbindungen im Sinne der vorliegenden Erfindung sind insbesondere die Ascorbylester der allgemeinen Struktur 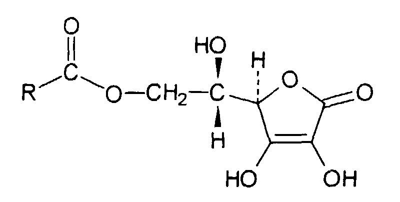 wobei R einen verzweigten oder unverzweigten Alkylrest mit bis zu 25 Kohlenstoffatomen darstellen kann.

Es war für den Fachmann nicht vorauszusehen gewesen, daß die erfindungsgemäß verwendeten kosmetischen oder dermatologischen Zubereitungen Ascorbinsäure und/oder Ascorbylverbindungen besser gegen den Zerfall stabilisieren als die Zubereitungen des Standes der Technik und daß sie ferner ausgezeichnete kosmetische und dermatologische Grundlagen für Ascorbinsäure und/oder Ascorbylverbindungen darstellen.

Zwar kennt der Stand der Technik die Verwendung von Ascorbinsäure in Emulsionen unter Verwendung von Siliconemulgatoren bei niedrigem pH-Wert (EP-B 670 157). Auch wird die Verwendung von Ascorbinsäure in wasserarmen Grundlagen (EP-A 770 381) bzw. Emulsionen, die einen hohen Gehalt an Polyolen aufweisen (EP-A 755 674) beschrieben. Ferner offenbart die WO-Schrift WO 98/00103-A1 als geeignete Grundlagen für eine Ascorbinsäureformulierung Wasser-in-Siliconöl-Systeme.

Allerdings ist diesen Schriften keinerlei Hinweis zu entnehmen, welcher in die Richtung der vorliegenden Erfindung weisen könnte.

Insbesondere in den O/W-Emulsionen des Standes der Technik zerfielen Vitamin C und seine Derivate unglücklicherweise sehr schnell. In der Publikation von R. Austria, A. Semenzato und A. Bettero (*J*. *Pham. Biomed. Anal*. *15 (1997) 795-801*) wird z. B. die Stabilität von Ascorbinsäure und einigen Derivaten in verschiedenen O/W-Emulsionen sowie in Lösung untersucht. Die Autoren stellten dabei einen Aktivitätsveilust von bis zu 60 % nach 2 Monaten bei Raumtemperatur in wässriger Lösung fest.

Es war daher besonders überraschend, daß die erfindungsgemäß verwendeten kosmetischen oder dermatologischen Zubereitungen Ascorbinsäure und/oder Ascorbylverbindungen in O/W-Emulsionen ausgezeichnet stabilisieren. Die Stabilität von Ascorbinsäure und/oder Ascorbylverbindungen in Öl-in-Wasser-Formulierungen läßt sich durch die erfindungsgemäße Verwendung gegenüber dem Stand der Technik erheblich steigern.

Ein besonders vorteilhafter partiell neutralisierter Ester von Monoglyceriden und/oder Diglyceriden gesättigter Fettsäuren im Sinne der vorliegenden Erfindung ist das Glycerylstearat. Ein weiterer vorteilhafter Ester ist das Glyceryllaurat.

Die Gesamtmenge an einem oder mehreren erfindungsgemäß verwendeten partiell neutralisierten Estern von Monoglyceriden und/oder Diglyceriden gesättigter Fettsäuren in den fertigen kosmetischen oder dermatologischen Zubereitungen wird vorteilhaft aus dem Bereich von 0,1 bis 10,0 Gew.-%, bevorzugt 0,5 bis 3,0 Gew.-% gewählt, bezogen auf das Gesamtgewicht der Zubereitungen.

Der oder die Ester von Polyethylenglykol mit verzweigten und/oder unverzweigten, gesättigten und/oder ungesättigten Fettsäuren einer Kettenlänge von 12 bis 40 Kohlenstoffatomen werden erfindungsgemäß vorteilhaft aus der Gruppe der PEG-Stearate gewählt. Besonders vorteilhaft sind PEG-30-Stearat, PEG-40-Stearat und PEG-100-Stearat.

Es ist vorteilhaft im Sinne der vorliegenden Erfindung, Polyethylenglykolether A und Polyethylenglykolether B unabhängig voneinander aus der Gruppe der Polyethylenglykol(n)cetylether, Polyethylenglykol(n)stearylether und Polyethylenglykol(n)cetylstearylether zu wählen, wobei n eine ganze Zahl darstellt, die den Durchschnittswert der Anzahl der Ethylenoxideinheiten angibt und aus dem Bereich von 2 bis 50 gewählt wird. Besonders bevorzugt sind Zubereitungen, in denen einer der beiden Polyethylenglykolether A oder B Polyethylenglykol(20)cetylstearylether (Ceteareth-20) oder Polyethylenglykol(2)stearylether (Steareth-2) ist.

Kosmetische und dermatologische Zubereitungen im Sinne der vorliegenden Erfindung enthalten vorzugsweise insgesamt 0,01 bis 20 Gew.-%, insbesondere 0,1 bis 10 Gew.-%, besonders bevorzugt 0,5 bis 6 Gew.-% der beiden Polyethylenglykolether A und B, jeweils bezogen auf das Gesamtgewicht der Zubereitungen. Vorteilhaft wird das Gewichtsverhältnis von Polyethylenglykolether A zu Polyethylenglykolether B in der Polyethylenglykolethermischung aus dem Bereich von 0,01 : 1 bis 1: 0,01 gewählt.

Bevorzugter, erfindungsgemäß verwendeter Fettalkohol ist der Cetyl-Stearylalkohol (ein Gemisch aus Hexadecanol-1 und Octadecanol-1 zu etwa gleichen Anteilen).

Die Gesamtmenge an einem oder mehreren erfindungsgemäß verwendeten Fettalkoholen in den fertigen kosmetischen oder dermatologischen Zubereitungen wird vorteilhaft aus dem Bereich von 0,1 bis 10,0 Gew.-%, bevorzugt 0,5 bis 6,0 Gew.-% gewählt, bezogen auf das Gesamtgewicht der Zubereitungen.

Bevorzugt stellen die erfindungsgemäßen Zubereitungen Emulsionen, insbesondere O/W-Emulsionen dar.

Die Ascorbylverbindung oder die Ascorbylverbindungen, insbesondere Vitamin C, ist bzw. sind erfindungsgemäß vorteilhaft in kosmetischen oder dermatologischen Zubereitungen bevorzugt zu 0,001 Gew.-% bis 10 Gew.-%, bevorzugt zu 0,05 Gew.-% bis 5 Gew.-%, insbesondere bevorzugt zu 0,1 bis 3,0 Gew.-%, bezogen auf das Gesamtgewicht der Zubereitungen, enthalten.

Die erfindungsgemäßen kosmetischen oder dermatologischen Zubereitungen enthalten ferner vorteilhaft einen oder mehrere Verdicker gewählt aus der Gruppe der Xanthangummies. Bevorzugt im Sinne der vorliegenden Erfindung ist Xanthan (CAS-Nr. 11138-66-2), welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen ( 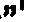 repeated units") besteht aus Glucose, Mannose, Glucuronsäure, Acetat und Pyruvat.

Die Gesamtmenge an einem oder mehreren Xanthangummies in den fertigen kosmetischen oder dermatologischen Zubereitungen wird vorteilhaft aus dem Bereich von 0,01 bis 0,5 Gew.-% gewählt, bezogen auf das Gesamtgewicht der Zubereitungen.

Die erfindungsgemäß verwendeten kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen.

Es ist erfindungsgemäß bevorzugt, den erfindungsgemäß verwendeten kosmetischen oder dermatologischen Zubereitungen Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner (Chelatoren) Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom oder -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz gewählt wird aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotnessigsäure (HOEDTA) und deren Anionen, Diethylenaminopentaessigsäure (DPTA) und deren Anionen, trans- 1,2-Diaminocyclohexantetraessigsäure (CDTA) und deren Anionen) sowie Natrium Chitosan Methylen Phosphonat.

Der oder die Komplexbildner sind erfindungsgemäß vorteilhaft in kosmetischen oder dermatologischen Zubereitungen bevorzugt zu 0,01 Gew.-% bis 15 Gew.-%, bevorzugt zu 0,05 Gew.-% bis 7 Gew.-%, insbesondere bevorzugt zu 0,1 bis 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O), eine multiple Emulsionen, beispielsweise vom Typ Wasser-in-Öl-in-Wasser (W/O/W), ein Gel, einen festen Stift, eine Salbe oder auch ein Aerosol darstellen. Erfindungsgemäß besonders vorteilhaft sind kosmetische und dermatologische Zubereitungen in Form von Öl-in-Wasser-Emulsionen mit mindestens einer wäßrigen Phase.

Es ist auch vorteilhaft, wenngleich selbstverständlich nicht zwingend, Ascorbinsäure und/oder Ascorbylverbindungen in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Besonders vorteilhafte Verkapselungsformen im Sinne der vorliegenden Erfindung sind ferner Cyclodextrinkomplexe von Vitamin C und/oder seinen Derivaten.

Es ist auch möglich und vorteilhaft im Sinne der vorliegenden Erfindung, erfindungsgemäß verwendete Zubereitungen in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Läsemittel oder Silikonderivate.

Insbesondere können erfindungsgemäß verwendete Zubereitungen auch mit anderen Antioxidantien und/oder Radikalfängern kombiniert werden.

Vorteilhaft werden solche Antioxidaritien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Sesamol, Sesamolin, Zink und dessen Derivate (z. B. ZnO, ZnSO₄) Seien und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew-%, insbesondere 0,1 bis 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß vorteilhaft sind Emulsionen, insbesondere O/W-Emulsionen; sie enthalten z. B. die genannten Fette, Öle, Wachse und anderen Fettkörper, sowie Wasser und gewünschtenfalls weitere Emulgatoren, wie sie üblicherweise für einen solchen Typ der Formulierung verwendet werden.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase wird im Sinne der vorliegenden Erfindung ferner vorteilhaft gewählt aus der Gruppe der Ester aus gesättigten und/oder ungesattigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, lsopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyctotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Gele gemaß der Erfindung enthalten üblicherweise Alkohole niedriger C-Zahl, z. B. Ethanol, lsopropanol, 1,2-Proparidiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei waßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Günstig sind auch solche kosmetischen und dermatologischen Zubereitungen, die in der Form eines Sonnenschutzmittels vorliegen. Vorzugsweise enthalten diese zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen enthalten, insbesondere der Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und demiatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen weitem Schutz der Zubereitungen selbst gegen Verderb dar.

Vorteilhaft enthalten erfindungsgemäße Zubereitungen Substanzen, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4 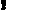 -methoxydibenzoylmethan (CAS-Nr. 70356-09-1), welches von Givaudan unter der Marke Parsol® 1789 und von Merck unter der Handeisbezeichnung Eusolex® 9020 verkauft wird.

Weitere vorteilhafte UV-A-Filtersubstanzen sind die Phenylen-1,4-bis-(2-benzimidazyl)-3,3 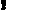 -5,5 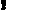 -tetrasulfonsäure: 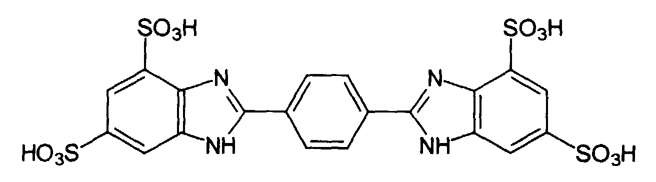 und ihre Salze, besonders die entsprechenden Natrium-, Kalium- oder Triethanolammonium-Salze, insbesondere das Phenylen-1,4-bis-(2-benzimidazyl)-3,3 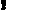 -5,5 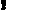 -tetrasulfonsäure-bis-natriumsalz: 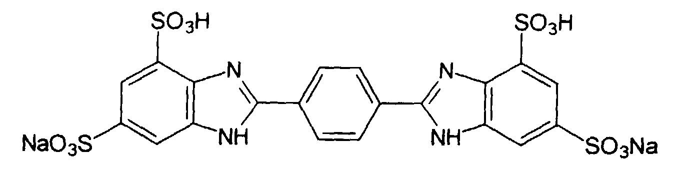 sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

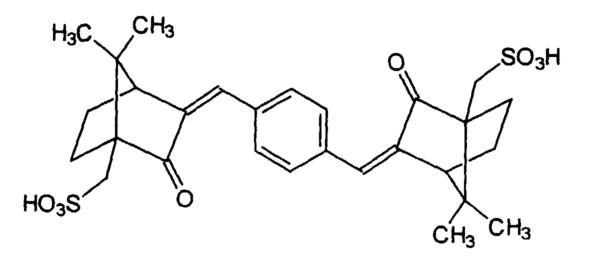

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter und/oder UV-B-Filtersubstanzen sind beispielsweise Bis-Resorcinyltriazinderivate mit der folgenden Struktur: 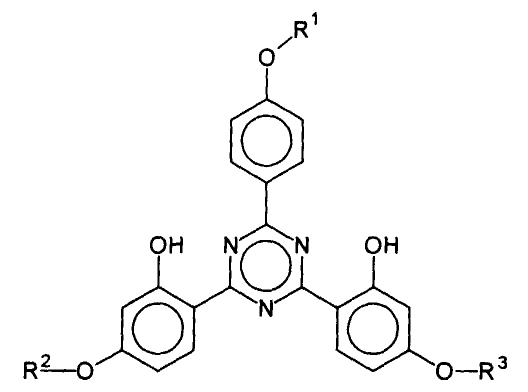 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin und das 4,4' 4''-(1,3,5-Triazin-2,4,6-triyltriimino)-tris-benzoesäure-tris(2-ethylhexylester), synonym: 2,4,6-Tris-[anilino-(p-carbo-2'-ethyl- 1'-hexyloxy)]- 1,3,5-triazin, welches von der BASF Aktiengesellschaft unter der Warenbezeichnung UVINUL® T 150 vertrieben wird.

Auch andere UV-Filtersubstanzen beschrieben, welche das Strukturmotiv 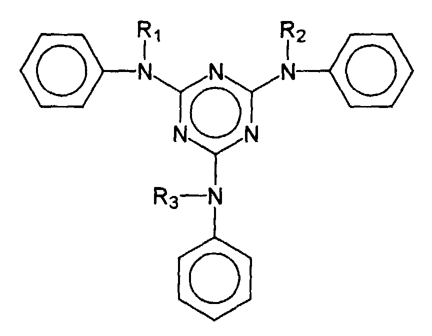 aufweisen sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 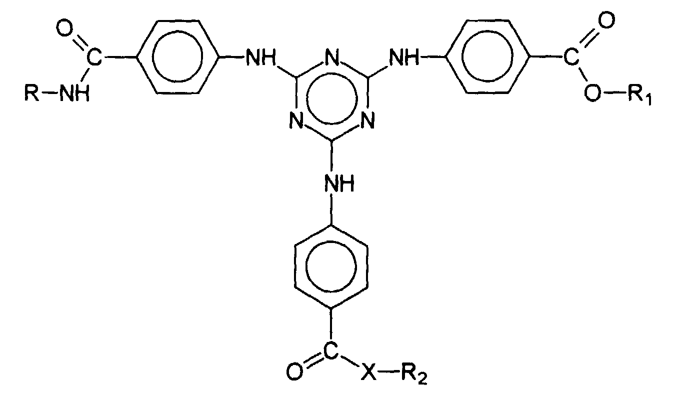 wiedergegeben wird, wobei
- R: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 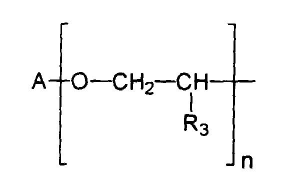 bedeutet, in welcher
- A: einen verzweigten oder unverzweigten C₁-C₁₈-Alkylrest, einen C₅-C₁₂-Cyclo-alkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 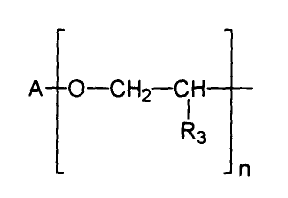 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Ein Beispiel für solche unsymmetrisch substituierte s-Triazine stellt das Dioctylbutylamidotriazon dar.

Auch andere UV-Filtersubstanzen, deren Einarbeitung in kosmetische oder dermatologische Lichtschutzformulierungen bisher Probleme aufwies, sind bekannt. So werden in der Europäischen Offenlegungsschrift 775 698 Bis-Resorcinyltriazinderivate beschrieben, deren chemische Struktur durch die generische Formel 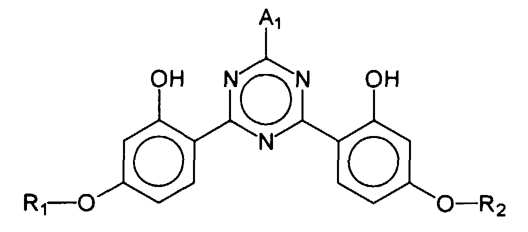 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(2-Ethyl-hexyloxy)-2-hydroxy]-phenyl}-6-(4-methoxyphenyl)- 1,3,5-triazin, das 2,4-Bis-{[4-(3-sulfonato)-2-hydroxy-propyloxy)-2-hydroxy]-phenyl}-6-(4-methoxyphenyl)- 1,3,5-triazin Natriumsalz, das 2,4-Bis-{[4-(3-(2-Propyloxy)-2-hydroxy-propyloxy)-2-hydroxy]-phenyl}-6-(4-methoxyphenyl)- 1,3,5-triazin, das 2,4-Bis-{[4-(2-ethyl-hexytoxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]- 1,3, 5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{(4-tris(trimethylsiloxy-silylpropyloxy)-2-hydroxy]-phenyl}-6-(4-methoxyphenyl)-1,3,5-triazin, das 2,4-Bis-{[4-(2 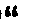 -methylpropenyloxy)-2-hydroxy]-phenyl}-6-(4-methoxyphenyl)-1,3,5-triazin und das 2,4-Bis-{[4-(1 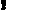 ,1 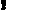 ,1 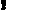 ,3 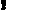 ,5 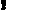 ,5 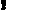 ,5 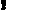 -Heptamethylsiloxy-2 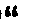 -methyl-propyloxy)-2-hydroxy]-phenyl}-6-(4-methoxyphenyl)-1,3,5-triazin.

Ein weiterer vorteilhafter UV-Filter im Sinne der vorliegenden Erfindung ist das 2,2 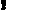 -Methylen-bis-(6-(2H-benzotriazol-2-yl)-4-(1,1,3,3-tetramethylbutyl)-phenol), welches durch die chemische Strukturformel 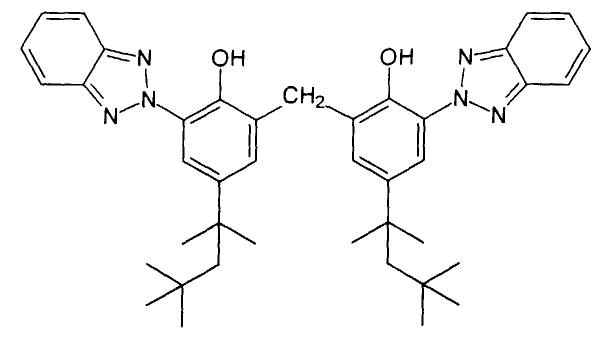 gekennzeichnet ist.

Vorteilhaft im Sinne der vorliegenden Erfindung ist ferner das 2-(2H-benzotriazol-2-yl)-4-methyl-6-(2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 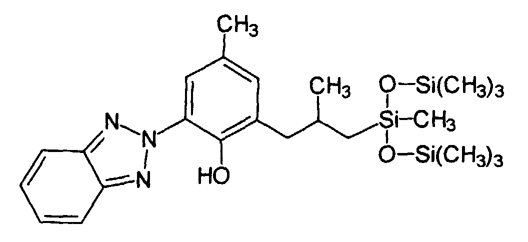 gekennzeichnet ist.

Die UV-B-Filter können öllöslich oder wasserlöslich sein. Vorteilhafte öllösliche UV-B-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Denvate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1, 3, 5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsaure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

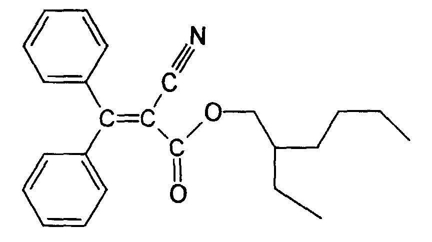

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäuredenvate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Gegenstand der Erfindung ist auch die Verwendung einer Kombination der erfindungsgemäß verwendeten Zubereitungen mit mindestens einem UV-A-Filter und/oder mindestens einem UV-B-Filter als Antioxidans bzw. die Verwendung einer Kombination der erfindungsgemäß verwendeten Zubereitungen mit mindestens einem UV-A-Filter und/oder mindestens einem UV-B-Filter als Antioxidans in einer kosmetischen oder dermatologischen Formulierung.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Fnsier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolytert sind z. B. wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch phamiazeutische oder kosmetische Unbedenklichkeit auszeichnen.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, die erfindungsgemäß verwendeten Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z. B. vor oder nach der Entfärbung, vor oder nach der Shampoonierung, zwischen zwei Shampoonierungsschritten, vor oder nach der Dauerwellbehandlung angewendet wird, so handelt es sich dabei z. B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäß verwendete Wirkstoffkombinationen in wirksamer Konzentration. Die Menge der verwendeten Polymeren liegt z. B. zwischen 0,1 und 10 Gew.-%, bevorzugt zwischen 0,1 und 3 Gew.-%.

Kosmetische Zubereitungen zur Behandlung und Pflege der Haare, die die erfindungsgemäß verwendeten Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an Isoquercitrin und dafür üblicherweise verwendeten Lösungsmitteln, bevorzugt Wasser, noch organische Verdickungsmittel, z. B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z. B. in einer Menge zwischen 0,1 und 30 Gew.-%, bevorzugt zwischen 0,5 und 15 Gew.-%, enthalten.

Vorzugsweise beträgt die Menge an Vitamin C in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z. B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglykolether-Derivate

Kosmetische Zubereitungen, die kosmetische Reinigungszubereitungen für die Haut darstellen, können in flüssiger oder fester Form vorliegen. Sie enthalten neben erfindungsgemäß verwendeten Zubereitungen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an Isoquercitrin vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfeftende Agentien, Komplexierungs- und Sequestrierungsagentien, Perlglanzagentien, Pflanzenextrakte, Vitamine, Wirkstoffe und dergleichen.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an erfindungsgemäß verwendeten Zubereitungen enthält, in ausreichender Menge auf die Haut oder Haare aufbringt.

Obwohl sich die erfindungsgemäß erzielten kosmetischen oder dermatologischen Zubereitungen durch erhöhte Stabilität gegenüber oxidativem Einfluß auszeichnen, sind doch Lagerungsformen bevorzugt, in welchen ein verminderter Zutritt von Luftsauerstoff gegeben ist. So ist beispielsweise die Befüllung unter Inertgas, insbesondere Stickstoff, vorteilhaft. Als vorteilhafte Verpackung haben sich insbesondere Aluminiumtuben sowie beschichtete, luftundurchlässige Kunststofftuben herausgestellt.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

| **Beispiel 1 (O/W-Creme):** | |
|---|---|
| | Gew.-% |
| Glycerylstearat | 2.00 |
| Ceteareth-20 | 1.50 |
| Steareth-2 | 1.00 |
| PEG-100 Stearat | 0.70 |
| Cetylstearylalkohol | 2.50 |
| Caprylsäure-/Caprinsäure Triglycerid | 3.00 |
| Octyldodecanol | 6.00 |
| Glycerin | 3.00 |
| Ascorbinsäure | 3.00 |
| Xanthangummi | 0.15 |
| Carbomer | 0.10 |
| EDTA | 0.10 |
| Natriumhydroxid | q.s. |
| Konservierung | q.s. |
| Parfum | q.s. |
| Wasser,demineralisiert | ad 100.0 |
| pH-Wert eingestellt auf 5.5 | |

| **Beispiel 2 (O/W-Lotion):** | |
|---|---|
| | Gew.-% |
| Glycerylstearat | 1.50 |
| Cetheareth-20 | 2.00 |
| Steareth-2 | 1.30 |
| PEG-100 Stearat | 0.80 |
| Cetylalkohol | 2.00 |
| Caprylsäure-/Caprinsäure Triglycerid | 3.00 |
| Dicaprylylether | 2,00 |
| Octyldodecanol | 1.00 |
| Glycerin | 5.00 |
| Ascorbinsäure | 1.00 |
| Xanthangummi | 0.15 |
| EDTA | 0.20 |
| Natriumhydroxid | q.s. |
| Konservierung | q.s. |
| Parfum | q.s. |
| Wasser,demineralisiert | ad 100.0 |
| pH-Wert eingestellt auf 5.5 | |

| **Beispiel 3 (O/W-Creme):** | |
|---|---|
| | Gew.-% |
| Glycerylstearat | 2.50 |
| Cetheareth-20 | 1.00 |
| Steareth-2 | 1.50 |
| PEG-30 Stearat | 1.50 |
| Stearylalkohol | 3.50 |
| Paraffin Oil | 3.00 |
| Caprylsäure-/Caprinsäure Triglycerid | 3,00 |
| Dicaprylylether | 2,00 |
| Ascorbinsäure | 5.00 |
| Xanthangummi | 0.15 |
| EDTA | 0.20 |
| Glycerin | 5,00 |
| Parfüm, Konservierungsmittel, Farbstoffe usw. | q.s. |
| Wasser | ad 100,00 |
| pH-Wert eingestellt auf 5.0 | |

| **Beispiel 4 (O/W-Creme):** | |
|---|---|
| | Gew. -% |
| Glycerylstearat | 2.00 |
| Cetheareth-20 | 1.50 |
| Steareth-2 | 0.50 |
| PEG-40 Stearat | 1.50 |
| Cetylalkohol | 3.00 |
| Paraffin Oil | 2.50 |
| Caprylsäure-/Caprinsäure Triglycerid | 3.00 |
| Octyldodecanol | 4.00 |
| Glycerin | 4.00 |
| Ascorbinsäure | 5.00 |
| Xanthangummi | 0.20 |
| EDTA | 0.20 |
| Natriumhydroxid | q.s. |
| Konservierung | q.s. |
| Parfum | q.s. |
| Wasser,demineralisiert | ad 100.0 |
| pH-Wert eingestellt auf 5.5 | |

| **Beispiel 5 (O/W-Creme):** | |
|---|---|
| | Gew.-% |
| Glycerylstearat | 2.50 |
| Cetheareth-20 | 1.50 |
| Steareth-2 | 1.00 |
| PEG-100 Stearat | 0.50 |
| Cetylstearylalkohol | 4.00 |
| Octyldodecanol | 3,00 |
| Caprylsäure-/Caprinsäuretriglycerid | 3,00 |
| Squalan | 1,00 |
| Jojobaöl | 1,00 |
| Cyclomethicon | 1,00 |
| Dimethicone | 0,50 |
| Vitamin E Acetat | 2.00 |
| Paraffinium liquium | 1,00 |
| Hydrierte Kokosfettsäureglyceride | 2,00 |
| Glycerin | 3,00 |
| Ascorbinsäure | 3.00 |
| Xanthangummi | 0,25 |
| Citroriensäure | 0,10 |
| Natriumcitrat | 0,3 0 |
| Parfüm, Konservierungsmittel, Farbstoffe | q.s. |
| Wasser | ad 100,00 |
| pH-Wert eingestellt auf 5.0 | |

| **Beispiel 6 (O/W-Creme):** | |
|---|---|
| | Gew.-% |
| Glycerylstearat | 1.50 |
| Cetheareth-20 | 2.00 |
| Steareth-2 | 1.00 |
| PEG-100 Stearat | 1.00 |
| Cetylstearylalkohol | 3.00 |
| Octyldodecanol | 2,00 |
| Caprylsäure-/Caprinsäuretriglycerid | 3,00 |
| Cyclomethicon | 2,00 |
| Paraffin Oil | 4.00 |
| Panthenol | 1.00 |
| Sorbitol | 2.00 |
| Tocopherylacetat | 1.00 |
| Ascorbinsäure | 3.00 |
| Carbomer | 0,10 |
| Xanthangummi | 0,15 |
| Parfüm, Konservierungsmittel, Farbstoffe usw. | q.s. |
| Wasser | ad 100,00 |
| pH-Wert eingestellt auf 6.5 | |

| **Beispiel 7 (Sonnenschutz-Creme):** | |
|---|---|
| | Gew.-% |
| Glycerylstearat | 3.00 |
| Cetheareth-20 | 2.50 |
| Steareth-2 | 0.50 |
| PEG-100 Stearat | 1.00 |
| Cetylstearylalkohol | 3,00 |
| Octyldodecanol | 3,00 |
| Paraffin Oil | 2.00 |
| Dicaprylylether | 3,00 |
| Caprylsäure-/Caprinsäuretriglycerid | 2,00 |
| Vitamin E Acetate | 2.00 |
| Xanthangummi | 0,15 |
| Natriumcarbomer | 0,10 |
| Glycerin | 3,00 |
| Octyltriazon | 2,00 |
| Methylbenzylidencampher | 3,00 |
| Butylmethoxydibenzoylmethan | 1.50 |
| Ascorbinsäure | 3.00 |
| Disodium EDTA | 0.20 |
| Parfüm, Konservierungsmittel, Farbstoffe, usw. | q.s. |
| Wasser | ad 100,00 |
| pH-Wert eingestellt auf 5.5 | |

| **Beispiel 8 (Emulgatorgel):** | |
|---|---|
| | Gew.-% |
| Glycerylstearat | 2.50 |
| Cetheareth-20 | 1.50 |
| Steareth-2 | 1.00 |
| PEG-100 Stearat | 0.50 |
| Cetylstearylalkohol | 1,50 |
| Ethanol | 2.00 |
| Ascorbinsäure | 2.00 |
| Aluminium Starch Octenylsuccinat | 0.25 |
| Xanthangummi | 0,15 |
| Disodium EDTA | 0.20 |
| Glycerin | 3,00 |
| Parfüm, Konservierungsmittel, Farbstoffe usw. | q.s. |
| Wasser | ad 100,00 |
| pH-Wert eingestellt auf 5.5 | |

## Patentansprüche

1. Verwendung von kosmetischen oder dermatologischen Zubereitungen, enthaltend
(I) einen oder mehrere partiell neutralisierte Ester von Monoglyceriden und/oder Diglyceriden gesättigter Fettsäuren,
(II) einen oder mehrere Ester von Polyethylenglykol mit verzweigten und/oder unverzweigten, gesättigten und/oder ungesättigten Fettsäuren einer Kettenlänge von 12 bis 40 Kohlenstoffatomen,
(III) mindestens zwei Polyethylenglykolether A und B, welche unabhängig voneinander gewählt werden aus der Gruppe der Ether von Polyethylenglykol mit verzweigten und/oder unverzweigten, gesättigten und/oder ungesättigten Fettalkoholen einer Kettenlänge von 16 bis 40 Kohlenstoffatomen, wobei sich die Kettenlänge des Ethers A um mindestens zwei Kohlenstoffatome von der Kettenlänge des Ethers B unterscheidet,
(IV) einen oder mehrere Fettalkohole, gewählt aus der Gruppe verzweigten und/oder unverzweigten, gesättigten und/oder ungesättigten Alkylalkohole mit 12 bis 40 Kohlenstoffatomen,
zur Stabilisierung von Ascorbinsäure und/oder Ascorbylverbindungen.

2. Verwendung nach Anspruch 1, dadurch gekennzeichnet, daß die kosmetische oder dermatologische Zubereitung in Form einer Öl-in-Wasser Emulsion vorliegt, welche mindestens eine wäßrige Phase enthält.

3. Verwendung nach Anspruch 1, dadurch gekennzeichnet, daß die kosmetische oder dermatologische Zubereitung zusätzlich Hils-, Zusatz- und/oder weitere Wirkstoffe und/oder Stabilisatoren enthält.

4. Verwendung nach Anspruch 1, dadurch gekennzeichnet, daß die kosmetische oder dermatologische Zubereitung 0,05 bis 0,5 Gew.-%, bezogen auf das Gesamtgewicht der Zubereitung, eines oder mehrerer Verdicker gewählt aus der Gruppe der Xanthan Gummies enthält.

5. Kosmetische und dermatologische Zubereitungen, enthaltend
(I) einen oder mehrere partiell neutralisierte Ester von Monoglyceriden und/oder Diglyceriden gesättigter Fettsäuren,
(II) einen oder mehrere Ester von Polyethylenglykol mit verzweigten und/oder unverzweigten, gesättigten und/oder ungesättigten Fettsäuren einer Kettenlänge von 12 bis 40 Kohlenstoffatomen,
(III) mindestens zwei Polyethylenglykolether A und B, welche unabhängig voneinander gewählt werden aus der Gruppe der Ether von Polyethylenglykol mit verzweigten und/oder unverzweigten, gesättigten und/oder ungesättigten Fettalkoholen einer Kettenlänge von 16 bis 40 Kohlenstoffatomen, wobei sich die Kettenlänge des Ethers A um mindestens zwei Kohlenstoffatome von der Kettenlänge des Ethers B unterscheidet,
(IV) einen oder mehrere Fettalkohole, gewählt aus der Gruppe verzweigten und/oder unverzweigten, gesättigten und/oder ungesättigten Alkylalkohole mit 12 bis 40 Kohlenstoffatomen und
(V) einen oder mehrere Verdicker, gewählt aus der Gruppe der Xanthangummies und
(VI) eine oder mehrere Verbindungen gewählt aus der Gruppe bestehend aus Ascorbinsäure und Ascorbylverbindungen.

6. Verwendung nach Anspruch 1 oder Zubereitung nach Anspruch 5, dadurch gekennzeichnet, daß die Ascorbinsäure und/oder die Ascorbylverbindungen (eine oder mehrere Verbindungen) in der Zubereitung in einer Konzentration von 0,01 bis 10 Gew.-%, bevorzugt 0,05 bis 5 Gew.-%, insbesondere 0,1 bis 3,0 Gew.-%, bezogen auf das Gesamtgewicht der Zubereitung, vorliegt.

7. Verwendung nach Anspruch 1 oder Zubereitung nach Anspruch 5, dadurch gekennzeichnet, daß die Ascorbinsäure und/oder die Ascorbylverbindungen (eine oder mehrere Verbindungen) in einem wirksamen Gehalt in der kosmetischen oder dermatologischen Zubereitung vorliegt.

8. Verwendung nach Anspruch 1 oder Zubereitung nach Anspruch 5, dadurch gekennzeichnet, daß als partiell neutralisierter Ester Glycerylstearat gewählt wird.

9. Verwendung nach Anspruch 3, dadurch gekennzeichnet, daß der oder die Stabilisatoren gewählt wird oder werden aus der Gruppe bestehend aus Polyolen (insbesondere Glycerin, Butylenglykol Sorbitol, Polyethylenglykol), Phytinsäure, Lactoferrin Natrium Chitosan Methylen Phosphonat, Mono- und Polysacchariden, Mono- und Polyphenolen, Weinsäure und deren Anionen, Citronensäure und deren Anionen, Aminopolycarbonsäuren und deren Anionen (insbesondere Ethylendiamintetraessigsäure und deren Anionen, Nitrilotriessigsäure und deren Anionen, Hydroxyethylendiaminotriessigsäure und deren Anionen, Diethylenaminopentaessigsäure und deren Anionen, trans-1,2-Diaminocyclohexantetraessigsäure und deren Anionen).

10. Verwendung nach Anspruch 3, dadurch gekennzeichnet, daß der oder die Stabilisatoren in der kosmetischen oder dermatologischen Zubereitung zu 0,01 Gew.-% bis 15 Gew.-%, bevorzugt zu 0,05 Gew.-% bis 10 Gew.-%, insbesondere bevorzugt zu 0,1 bis 5,0 Gew.-%, bezogen auf das Gesamtgewicht der Zubereitungen, enthalten ist oder sind.

11. Zubereitung nach Anspruch 5, dadurch gekennzeichnet, daß der Gehalt an Verdicker (eine oder mehrere Verbindungen) gewählt wird aus dem Bereich 0,05 bis 0.5 Gew.-%, bezogen auf das Gesamtgewicht der Zubereitung.
